(19) Europäisches Patentamt
European Patent Office
Office européen des brevets

(11) EP 0 966 434 B1

(12) **FASCICULE DE BREVET EUROPEEN**

(45) Date de publication et mention de la délivrance du brevet:
**22.05.2002 Bulletin 2002/21**

(21) Numéro de dépôt: **98942789.3**

(22) Date de dépôt: **26.08.1998**

(51) Int Cl.[7]: **C07C 271/28**, C07C 275/34, A61K 7/48

(86) Numéro de dépôt international:
**PCT/FR98/01856**

(87) Numéro de publication internationale:
**WO 99/10318 (04.03.1999 Gazette 1999/09)**

(54) **DERIVES D'AMINOPHENOL ET LEUR UTILISATION EN COSMETIQUE**

AMINOPHENOLDERIVATE UND IHRE VERWENDUNG IN DER KOSMETIK

AMINOPHENOL DERIVATIVES AND THEIR USE IN COSMETICS

(84) Etats contractants désignés:
**AT BE CH DE ES FR GB IT LI NL SE**

(30) Priorité: **27.08.1997 FR 9710710**

(43) Date de publication de la demande:
**29.12.1999 Bulletin 1999/52**

(73) Titulaire: **L'OREAL**
**75008 Paris (FR)**

(72) Inventeurs:
- **PHILIPPE, Michel**
  **F-91320 Wissous (FR)**
- **TULOUP, Rémy**
  **F-75014 Paris (FR)**
- **BLAISE, Christian**
  **F-94160 Saint Mande (FR)**

(74) Mandataire: **Renard, Emmanuelle**
**L'OREAL-DPI**
**6 rue Bertrand Sincholle**
**92585 Clichy Cedex (FR)**

(56) Documents cités:
**DD-A- 107 449**
**FR-A- 2 756 734**
**GB-A- 1 205 029**
**US-A- 2 663 730**
**US-A- 3 933 470**

- **DATABASE WPI Week 9722 Derwent Publications Ltd., London, GB; AN 97-241638 XP002086051 "Skin external agents used for whitening skin - contains 4-aminophenol and eg. polyoxyethylene" & JP 09 077655 A (MIKIMOTO SEIYAKU) , 25 mars 1997**
- **A. TIENNE ET AL: "Isocyanurates diméthylés-1.3 et arylés-5" COMPTES RENDUS HEBDOMADAIRES DES SEANCES DE L'ACADEMIE DES SCIENCES, SERIE C: SCIENCES CHIMIQUES., vol. 279, 2 décembre 1974, pages 969-972, XP000602110 MONTREUIL FR**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à l'utilisation de dérivés d'aminophénol comme agents dépigmentants ou blanchissants dans une composition cosmétique et/ou dermatologique, à une composition cosmétique et/ou dermatologique comprenant ces dérivés et à de nouveaux dérivés d'aminophénol.

La couleur de la peau humaine est fonction de différents facteurs et notamment des saisons de l'année, de la race et du sexe, et elle est principalement déterminée par la nature et la concentration de mélanine produite par les mélanocytes. Les mélanocytes sont les cellules spécialisées qui par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus clairs. Ceci est particulièrement intéressant pour les poils qui sont moins visibles lorsqu'ils sont clairs que lorsqu'ils sont foncés.

Le mécanisme de formation de la pigmentation de la peau, des poils et des cheveux, c'est-à-dire de la formation de la mélanine est particulièrement complexe et fait intervenir schématiquement les principales étapes suivantes :

$$\text{Tyrosine} \rightarrow \text{Dopa} \rightarrow \text{Dopaquinone} \rightarrow \text{Dopachrome} \rightarrow \text{Mélanine}$$

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé peut provoquer des réactions d'allergie ("Contact allergy to kojic acid in skin care products", Nakagawa M. et al., in Contact Dermatitis, Jan. 95, Vol 42 (1), pp.9-13). Ce composé est également instable en solution, ce qui complique quelque peu la fabrication de la composition.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Aussi, il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau et stable dans une composition.

La demanderesse a trouvé de manière inattendue que des dérivés d'aminophénol présentent une activité dépigmentante, même à faibles concentrations, sans faire preuve de cytotoxicité.

Certains de ces dérivés sont déjà connus de US-A-2 663 730, US-A-3 933 470, GB-A-1 205 029, DD 107 449, A. ETIENNE ET AL « Isocyanurates diméthylés-1.3 et arylés-5 », Comptes rendus hebdomadaires des séances de l'académie des sciences, Série C : sciences chimiques, Vol. 279, 2 décembre 1974, pp. 969-972, JP-09 077 655, ou de FR-A-2 756 734 par exemple. D'autres dérivés d'aminophénol ont été découverts par la Demanderesse.

La présente invention a donc pour objet l'utilisation d'au moins un dérivé d'aminophénol de formule (I) suivante :

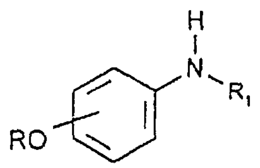

(I)

dans laquelle :

R représente un atome d'hydrogène ou un radical $-COR_2$,
avec $R_2$ représentant un radical choisi parmi un radical alkyle ou alkoxyle, saturé ou insaturé, linéaire, cyclique ou ramifié; en $C_1$ à $C_{30}$, éventuellement hydroxylé,

$R_1$ est choisi parmi un radical de formules (a), (b) ou (c) suivantes :

(a)$-CO-NR_3R_4$
(b) $-CO-O-R_5$
(c)$-SO_2-R_5$
avec $R_3$ représentant un atome d'hydrogène ou un radical alkyle en $C_1$ à $C_6$, linéaire ou ramifié, saturé ou insaturé, éventuellement hydroxylé,
avec $R_4$ représentant un atome d'hydrogène ou un radical choisi parmi un radical alkyle, saturé ou insaturé, linéaire, cyclique ou ramifié, en $C_1$ à $C_{30}$, éventuellement hydroxylé,
avec $R_5$ représentant un radical choisi parmi un radical alkyle, saturé ou insaturé, linéaire, cyclique ou ramifié, en $C_1$ à $C_{30}$, éventuellement hydroxylé,

dans une composition cosmétique dépigmentante et/ou blanchissante de la peau humaine, des poils ou des cheveux.

Ces composés présentent l'avantage d'être faciles à obtenir. Ils peuvent être notamment obtenus en faisant réagir un aminophénol avec une entité chimique activée, telle qu'un imidazolide ou un isocyanate, lorsque $R_1$ est un radical de formule (a), un chloroformiate, lorsque $R_1$ est un radical de formule (b), ou un chlorure de sulfonyle, lorsque $R_1$ est un radical de formule (c).

Selon la présente invention, parmi les radicaux alkyle linéaire ou ramifié ayant de 1 à 30 atomes de carbone, on peut citer avantageusement les radicaux méthyle, éthyle, propyle, isopropyle, butyle, tertiobutyle, hexyle, octyle, nonyle, 2-éthyl-hexyl et dodécyle. De préférence, ces radicaux présentent de 1 à 12 atomes de carbone. De manière encore plus préférentielle, le radical alkyle comprend généralement de 1 à 6 atomes de carbone. On peut citer, comme radical alkyle inférieur, les radicaux méthyle, éthyle, propyle, isopropyle, tertiobutyle, hexyle.

Parmi les radicaux alkyle linéaire ayant de 1 à 30 atomes de carbone, on peut citer notamment les radicaux méthyle, éthyle, propyle, octyle, dodécyle, tridécyle, hexadécyle, béhényle, octadécyle, tétracosyl, hexacosyl, octacosyl et myricyl.

Parmi les radicaux alkyle ramifié ayant de 1 à 30 atomes de carbone, on peut citer notamment les radicaux 2-éthyl-hexyle, 2-butyl-octyle, 2-hexyl-décyle.

Lorsqu'il est insaturé, on préfère un radical présentant une ou plusieurs insaturations éthyléniques, tel que les radicaux neryl, 2-nonyl 2-butényl, 6-(1,3-pentadiényl)-2,4,7-dodécanetrièn-9-ynyl et plus particulièrement le radical allyle.

Lorsque le radical alkyle est cyclique, on peut notamment citer le radical cyclohexyle, cholestéryle ou terbutylcyclohexyle.

Lorsqu'il est hydroxylé, le radical comprend de préférence 1 à 6 atomes de carbone et 1 à 5 groupes hydroxyles.

Parmi les radicaux monohydroxyalkyle, on préfère un radical contenant de préférence 1 à 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle et 2,3,4,5,6-pentahydroxyhexyle.

Les radicaux alkoxylés sont des radicaux alkyles, tels que notamment décrits ci-dessus, précédés d'un atome d'oxygène.

De préférence, les dérivés d'aminophénol de la présente invention sont ceux pour lesquels l'une au moins et de préférence toutes les conditions ci-dessous sont respectées :

- R représente un atome d'hydrogène,
- la fonction -OR sur le radical phényl est en position ortho ou avantageusement en position para,
- $R_1$ est choisi parmi un radical de formules (a) ou (b).

Les composés de formule (I) sont plus particulièrement choisis parmi le N-éthyloxycarbonyl-4-amino-phénol ; le N-éthyloxycarbonyl-O-éthyloxycarbonyl-4-aminophénol ; N-cholestéryloxycarbonyl-4-amino-phénol et le N-éthylaminocarbonyl-4-aminophénol.

La présente invention a également pour objet des compositions cosmétiques et/ou dermatologiques comprenant au moins un dérivé d'aminophénol de formule (I) et un milieu cosmétiquement et/ou dermatologiquement acceptable. Cette composition est plus particulièrement destinée à un usage topique sur la peau et/ou ses phanères (cheveux, poils et ongles).

Cette composition cosmétique ou dermatologique est avantageusement destinée à dépigmenter et/ou blanchir la peau humaine et/ou enlever les taches pigmentaires de la peau et/ou dépigmenter les poils et/ou les cheveux.

La présente invention a aussi pour objet l'utilisation de ces dérivés d'aminophénol dans et/ou pour la fabrication d'une composition cosmétique et/ou dermatologique, comme inhibiteur de la tyrosinase et/ou de la synthèse de la mélanine et/ou comme agent dépigmentant et/ou blanchissant de la peau, des poils ou des cheveux.

La présente invention a aussi pour objet de nouveaux dérivés d'aminophénol de formule (I) suivante :

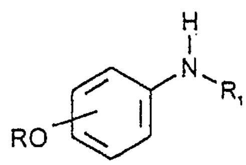

(I)

dans laquelle :

R représente un atome d'hydrogène ou un radical $-COR_2$,

avec $R_2$ représentant un radical choisi parmi un radical alkyle ou alkoxyle, saturé ou insaturé, linéaire, cyclique ou ramifié, en $C_1$ à $C_{30}$, éventuellement hydroxylé,

$R_1$ est choisi parmi les radicaux suivants :

(a) un radical $-CO-NR_3R_4$
(b) un radical cholestéryloxycarbonyl
(c) un radical $-SO_2-R_5$

avec $R_3$ représentant un atome d'hydrogène ou un radical alkyle en $C_1$ à $C_6$, linéaire ou ramifié, saturé ou insaturé, éventuellement hydroxylé,

avec $R_4$ représentant un radical choisi parmi un radical alkyle, saturé ou insaturé, linéaire, cyclique ou ramifié, en $C_{13}$ à $C_{30}$, éventuellement hydroxylé, et

avec $R_5$ représentant un radical choisi parmi un radical alkyle, saturé ou insaturé, linéaire, cyclique ou ramifié, en $C_1$ à $C_{30}$, éventuellement hydroxylé.

La présente invention se rapporte également à un procédé cosmétique de dépigmentation et/ou de blanchiment de la peau humaine, des poils ou des cheveux consistant à appliquer sur la peau, les poils ou les cheveux une

composition cosmétique selon l'invention.

La composition selon l'invention est appropriée pour une utilisation topique et contient donc un milieu cosmétiquement ou dermatologiquement acceptable, c'est-à-dire compatible avec la peau, les poils ou les cheveux.

Les dérivés d'aminophénol seront utilisés en une quantité efficace pour obtenir l'effet dépigmentant ou blanchissant désiré et cette quantité sera fonction de la nature des dérivés d'aminophénol considérés. En particulier, les dérivés d'aminophénol de formule (I) peuvent être notamment présents dans la composition en une quantité allant de 0,001 à 10 % et de préférence de 0,005 à 5 % du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse.

Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. On peut également associer les dérivés d'aminophénol à d'autres agents dépigmentants, tels que l'acide kojique ou l'hydroquinone et ses dérivés, ce qui permet d'utiliser ces derniers à des doses moins toxiques pour la peau. En cas d'incompatibilité, ces actifs et/ou les dérivés d'aminophénol peuvent être incorporés dans des sphérules, notamment des vésicules ioniques ou non-ioniques et/ou des nanoparticules (nanocapsules et/ou nanosphères), de manière à les isoler les uns des autres dans la composition.

L'invention va maintenant être illustrée à l'aide des exemples qui suivent. Les concentrations sont données en pourcentage en poids.

**Exemples de composés**

**Exemple 1**

le N-éthyloxycarbonyl-4-amino-phénol

20g de 4-amino-phénol (Mw = 109,13 ; 0,18324 mole) sont mis en suspension dans 200 cm3 de dichlorométhane anhydre, sous atmosphère inerte. On refroidit à une température de 0°C dans un bain de glace, puis on ajoute 16,4 cm3 de pyridine anhydre (Mw =79,10 ; d= 0,981 ; 0,2034 mole). Ensuite on ajoute, goutte à goutte, de manière à maintenir à une température inférieure à 10°C; 17,6 cm3 de chloroformiate d'éthyle (Mw = 108,53 ; d = 1,13 ; 0,18324 mole). On maintient le mélange réactionnel sous agitation pendant deux heures. On verse le mélange sur un litre d'eau glacée, puis on extrait à l'acétate d'éthyle. On lave la phase organique à l'eau puis on sèche, filtre et évapore sous vide. Après recristallisation dans un mélange heptane-acétate d'éthyle, on obtient une poudre blanche de masse 21g, ce qui correspond à un rendement de 63%. La température de fusion du produit obtenu est de 127,2°C. Le spectre RMN 1H et l'analyse élémentaire sont conformes au produit attendu.

**Exemple 2**

N-éthyloxycarbonyl-O-éthyloxycarbonyl-4-amino-phénol

10g de 4-amino-phénol (Mw = 109,13 ; 0,0916 mole) sont mis en suspension dans 100 cm3 de dichlorométhane anhydre, sous atmosphère inerte. On refroidit à une température de 0°C dans un bain de glace, puis on ajoute 8,2 cm3 de pyridine anhydre (Mw=79,10 ; d= 0,981 ; 0,102 mole). Ensuite on ajoute, goutte à goutte, de manière à maintenir à une température inférieure à 10°C; 9,8 cm3 de chloroformiate d'éthyle (Mw = 108,53 ; d = 1,13 ; 0,104 mole). On maintient le mélange réactionnel sous agitation pendant deux heures. On verse le mélange sur un litre d'eau glacée, puis on extrait à l'acétate d'éthyle. On lave la phase organique à l'eau puis on sèche, filtre et évapore sous vide. Après recristallisation dans l'éthanol, on obtient une poudre blanche de masse 18g, ce qui correspond à un rendement de 80%. La température de fusion du produit obtenu est de 110,8°C. Le spectre RMN 1H 200MHz et l'analyse élémentaire sont conformes au produit attendu.

**Exemple 3**

N-cholestéryloxycarbonyl-4aminophénol

21,8g de 4-amino-phénol (Mw = 109,13 ; 0,2 mole) sont mis en suspension dans 200 cm3 de N,N-diméthylacétamide anhydre, sous atmosphère inerte. Ensuite on ajoute, goutte à goutte, de manière à maintenir à une température inférieure à 30°C; 44,9 g de chloroformiate de cholestéryle (Mw = 449,11 ; 0,1 mole). On maintient le mélange réactionnel sous agitation pendant deux heures. On verse le mélange sur cinq litres d'eau, puis on filtre et on sèche le précipité.. Après recristallisation dans un mélange eau-éthanol, on obtient une poudre blanche de masse 46 g, ce qui correspond à un rendement de 88%. La température de fusion du produit obtenu est de 186,9°C. Le spectre RMN 13C 100MHz et l'analyse élémentaire sont conformes au produit attendu.

**Exemple 4**

N-Ethylaminocarbonyl-4aminophénol

10 g de 4-amino-phénol (Mw = 109,13 ; 0,0916 mole) sont mis en suspension dans 100 cm3 de N-méthylpyrrolidone anhydre, sous atmosphère inerte. Ensuite on ajoute, goutte à goutte, 6,5 g d'isocyanate d'éthyle (Mw = 71,08 ; 0,0914 mole). On porte le mélange réactionnel à une température de 60°C sous agitation pendant deux heures. On verse le mélange sur un litre d'eau, puis on filtre et on sèche le précipité.. Après recristallisation dans l'éthanol, on obtient une poudre blanche de masse 9 g, ce qui correspond à un rendement de 55%. La température de fusion du produit obtenu est de 173,4°C. Le spectre RMN 1H 200MHz et l'analyse élémentaire sont conformes au produit attendu.

**Tests :**

Un test biologique a mis en évidence l'activité dépigmentante des dérivés d'aminophénol de formule (I)

Ce test correspond à celui décrit dans le brevet FR 2734825 déposé par la Demanderesse, ainsi que dans l'article de R. Schmidt, P. Krien et M. Régnier, Anal. Biochem., 235(2), 113-18, (1996). Ce test est ainsi réalisé sur

coculture de kératinocytes et de mélanocytes.

Pour chaque composé testé, il est déterminé la valeur de IC50 qui correspond à la concentartion micromolaire (µM) pour laquelle est observée 50% d'inhibition de la mélanogénèse.

Par ailleurs, une classe est donnée à chacun de ces composés pour leur activité dépigmentante maximale :

classe 1 : 10 à 30% d'inhibition de la mélanogénèse par rapport au témoin (même expérience sans composé à tester) ;
classe 2 : 30 à 60% d'inhibition de la mélanogénèse par rapport au témoin (même expérience sans composé à tester) ;
classe 3 : 60 à 100% d'inhibition de la mélanogénèse par rapport au témoin (même expérience sans composé à tester).

Les résultats sont rassemblés dans le tableau (1) suivant.

| | IC 50 (µM) | Classe |
|---|---|---|
| Composé de l'exemple 1 | 1 | 3 à 1 µM |
| Composé de l'exemple 2 | 50 | 3 à 200 µM |
| Composé de l'exemple 3 | 10 | 3 à 200 µM |
| Acide kojique | 500 | 2 à 500 µM |

Ces composés de formule (1) présentent donc une plus grande efficacité dépigmentante que l'acide kojique. En outre, ils ont l'avantage de ne pas présenter de cytotoxicité à l'égard des kératinocytes et les mélanocytes, défaut majeur des dépigmentants existants.

## Exemples de compositions

### Exemple 5 : Crème traitante

- Alcool cétylique 1,05 %
- Stéarate de PEG-20 (Myrj 49 vendu par la société ICI) 2 %
- Cyclométhicone 6 %
- Composé de l'exemple 1 0,5 %
- Carbomer 0,6 %
- Glycérine 3 %
- Triéthanolamine 1 %
- Conservateurs 0,5 %
- Eau déminéralisée qsp 100 %

La crème obtenue utilisée en application quotidienne, permet d'obtenir un blanchiment de la peau.

### Exemple 6 : Gel traitant

- Propylène glycol 10 %
- Alcool éthylique 40 %
- Glycérine 3 %
- Composé de l'exemple 2 0,5 %
- Conservateurs 0,15 %
- Parfum 0,15 %
- Eau déminéralisée qsp 100 %

Le gel obtenu peut être utilisé quotidiennement et est apte à dépigmenter la peau.

**Exemple 7 : Stick traitant**

- Cire de Carnauba 5 %
- Ozokerite 7 %
- Lanoline 6 %
- Dioxyde de titane (pigments) 20 %
- Amidon de riz (charge) 7 %
- EDTA 0,1 %
- Composé de l'exemple 3 2 %
- Perhydrosqualène qsp 100 %

Le stick obtenu, utilisé sur les taches pigmentaires, permet de les atténuer voire de les faire disparaître.

## Revendications

1. Utilisation d'au moins un dérivé d'aminophénol de formule (I) suivante :

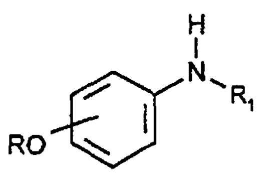

(I)

dans laquelle :

R représente un atome d'hydrogène ou un radical $-COR_2$,
avec $R_2$ représentant un radical choisi parmi un radical alkyle ou alkoxyle, saturé ou insaturé, linéaire, cyclique ou ramifié, en $C_1$ à $C_{30}$, éventuellement hydroxylé,

$R_1$ est choisi parmi un radical de formules (a), (b) ou (c) suivantes :

(a) $-CO-NR_3R_4$
(b) $-CO-O-R_5$
(c) $-SO_2-R_5$

avec $R_3$ représentant un atome d'hydrogène ou un radical alkyle en $C_1$ à $C_6$, linéaire ou ramifié, saturé ou insaturé, éventuellement hydroxylé,
avec $R_4$ représentant un atome d'hydrogène ou un radical choisi parmi un radical alkyle, saturé ou insaturé, linéaire, cyclique ou ramifié, en $C_1$ à $C_{30}$, éventuellement hydroxylé,
avec $R_5$ représentant un radical choisi parmi un radical alkyle, saturé ou insaturé, linéaire, cyclique ou ramifié, en $C_1$ à $C_{30}$, éventuellement hydroxylé,

dans une composition cosmétique dépigmentante et/ou blanchissante de la peau humaine, des poils ou des cheveux.

2. Utilisation selon la revendication 1, **caractérisée en ce que** ledit dérivé d'aminophénol présente l'une au moins des conditions ci-dessous :

- R représente un atome d'hydrogène,

- la fonction -OR sur le radical phényl est en position ortho ou en position para,
- $R_1$ est choisi parmi un radical de formules (a) ou (b).

3. Utilisation selon la revendication 1 ou 2, **caractérisée en ce que** ledit dérivé d'aminophénol est choisi parmi le N-éthyloxycarbonyl-4-amino-phénol ; le N-éthyloxycarbonyl-O-éthyloxycarbonyl-4-amino-phénol ; N-cholestéryloxycarbonyl-4-amino-phénol et le N-éthylaminocarbonyl-4-amino-phénol.

4. Utilisation d'au moins un dérivé d'aminophénol présentant la formule (I), tel que défini dans l'une des revendications 1 à 3, dans et/ou pour la fabrication d'une composition cosmétique et/ou dermatologique, comme inhibiteur de la tyrosinase et/ou de la synthèse de la mélanine.

5. Composition cosmétique et/ou dermatologique comprenant au moins un dérivé d'aminophénol de formule (I) tel que défini à l'une des revendications 1 à 4 et un milieu cosmétiquement et/ou dermatologiquement acceptable.

6. Composition selon la revendication précédente, **caractérisée en ce qu'**elle est destinée à dépigmenter et/ou blanchir la peau humaine et/ou enlever les taches pigmentaires de la peau et/ou dépigmenter les poils et/ou les cheveux.

7. Composition selon l'une des revendications 5 à 6, **caractérisée en ce que** le dérivé d'aminophénol est présent en une quantité allant de 0,01 à 10 % du poids total de la composition.

8. Composition selon l'une quelconque des revendications 5 à 7, **caractérisée en ce que** la composition comprend en outre, au moins un actif choisi parmi les agents kératolytiques et/ou desquamants, anti-inflammatoires, apaisants, autres agents dépigmentants et leurs mélanges.

9. Dérivés d'aminophénol de formule (I) suivante :

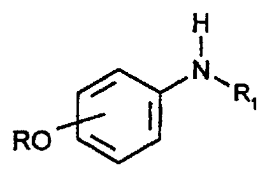

(I)

dans laquelle :

R représente un atome d'hydrogène ou un radical $-COR_2$,
avec $R_2$ représentant un radical choisi parmi un radical alkyle ou alkoxyle, saturé ou insaturé, linéaire, cyclique ou ramifié, en $C_1$ à $C_{30}$, éventuellement hydroxylé,

$R_1$ est choisi parmi les radicaux suivants :

(a) un radical $-CO-NR_3R_4$
(b) un radical cholestéryloxycarbonyl
(c) un radical $-SO_2-R_5$

avec $R_3$ représentant un atome d'hydrogène ou un radical alkyle en $C_1$ à $C_6$, linéaire ou ramifié, saturé ou insaturé, éventuellement hydroxylé,
avec $R_4$ représentant un radical choisi parmi un radical alkyle, saturé ou insaturé, linéaire, cyclique ou ramifié, en $C_{13}$ à $C_{30}$, éventuellement hydroxylé, et.
avec $R_5$ représentant un radical choisi parmi un radical alkyle, saturé ou insaturé, linéaire, cyclique ou ramifié, en $C_1$ à $C_{30}$, éventuellement hydroxylé

10. Procédé cosmétique de dépigmentation et/ou blanchiment de la peau humaine, des poils ou des cheveux, **caractérisé en ce qu'**il consiste à appliquer sur la peau, les poils ou les cheveux une composition décrite selon l'une des revendications précédentes 5 à 8.

**11.** Composition selon la revendication 7, **caractérisée en ce que** le dérivé d'aminophénol est présent en une quantité allant de 0,005 à 5 % du poids total de la composition.

## Claims

**1.** Use of at least one aminophenol derivative of formula (I) below:

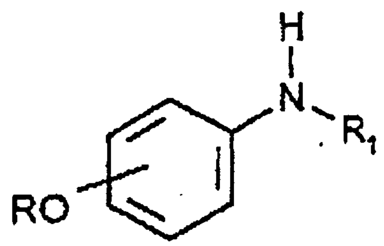

(I)

in which:

R represents a hydrogen atom or a radical $-COR_2$,

with $R_2$ representing a radical chosen from a saturated or unsaturated, linear, cyclic or branched, optionally hydroxylated, $C_1$ to $C_{30}$ alkyl or alkoxy radical,

$R_1$ is chosen from a radical of formula (a), (b) or (c) below:

(a) $-CO-NR_3R_4$
(b) $-CO-O-R_5$
(c) $-SO_2-R_5$

with $R_3$ representing a hydrogen atom or a linear or branched, saturated or unsaturated, optionally hydroxylated, $C_1$ to $C_6$ alkyl radical,

with $R_4$ representing a hydrogen atom or a radical chosen from a saturated or unsaturated, linear, cyclic or branched, optionally hydroxylated, $C_1$ to $C_{30}$ alkyl radical,

with $R_5$ representing a radical chosen from a saturated or unsaturated, linear, cyclic or branched, optionally hydroxylated, $C_1$ to $C_{30}$ alkyl radical,
in a depigmenting and/or bleaching cosmetic composition for human skin, body hairs or head hair.

**2.** Use according to Claim 1, **characterized in that** the said aminophenol derivative meets at least one of the conditions below:

- R represents a hydrogen atom,
- the function -OR on the phenyl radical is in an ortho position or in the para position,
- $R_1$ is chosen from a radical of formula (a) or (b).

**3.** Use according to Claim 1 or 2, **characterized in that** the said aminophenol derivative is chosen from N-ethyloxycarbonyl-4-aminophenol; N-ethyloxycarbonyl-O-ethyloxycarbonyl-4-aminophenol; N-cholesteryloxycarbonyl-4-aminophenol and N-ethylaminocarbonyl-4-aminophenol.

**4.** Use of at least one aminophenol derivative of formula (I), as defined in one of Claims 1 to 3, in and/or for the manufacture of a cosmetic and/or dermatological composition, as a tyrosinase inhibitor and/or as an inhibitor of

melanin synthesis.

**5.** Cosmetic and/or dermatological composition comprising at least one aminophenol derivative of formula (I) as defined in one of Claims 1 to 4 and a cosmetically and/or dermatologically acceptable medium.

**6.** Composition according to the preceding claim, **characterized in that** it is intended for depigmenting and/or bleaching human skin and/or for removing pigmentation marks from the skin and/or for depigmenting body hairs and/or head hair.

**7.** Composition according to either of Claims 5 and 6, **characterized in that** the aminophenol derivative is present in an amount ranging from 0.01 to 10% of the total weight of the composition.

**8.** Composition according to any one of Claims 5 to 7, **characterized in that** the composition also comprises at least one active agent chosen from keratolytic agents and/or desquamating agents, anti-inflammatory agents, calmants, other depigmenting agents and mixtures thereof.

**9.** Aminophenol derivatives of formula (I) below:

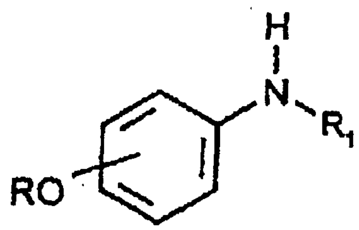

(I)

in which:

R represents a hydrogen atom or a radical $-COR_2$, with $R_2$ representing a radical chosen from a saturated or unsaturated, linear, cyclic or branched, optionally hydroxylated, $C_1$ to $C_{30}$ alkyl or alkoxy radical,

$R_1$ is chosen from the following radicals:

(a) a radical $-CO-NR_3R_4$
(b) a cholesteryloxycarbonyl radical
(c) a radical $-SO_2-R_5$

with $R_3$ representing a hydrogen atom or a linear or branched, saturated or unsaturated, optionally hydroxylated, $C_1$ to $C_6$ alkyl radical,

with $R_4$ representing a radical chosen from a saturated or unsaturated, linear, cyclic or branched, optionally hydroxylated, $C_{13}$ to $C_{30}$ alkyl radical, and

with $R_5$ representing a radical chosen from a saturated or unsaturated, linear, cyclic or branched, optionally hydroxylated, $C_1$ to $C_{30}$ alkyl radical.

**10.** Cosmetic process for depigmenting and/or bleaching human skin, body hairs or head hair, **characterized in that** it consists in applying a composition described according to one of Claims 5 to 8 above to the skin, body hairs or head hair.

**11.** Composition according to Claim 7, **characterized in that** the aminophenol derivative is present in an amount ranging from 0.005 to 5% of the total weight of the composition.

**Patentansprüche**

1. Verwendung mindestens eines Aminophenolderivats der Formel (I):

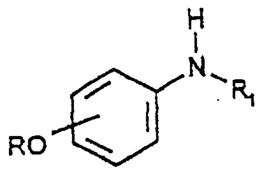

(I),

worin bedeuten:

- R Wasserstoff oder eine Gruppe $-COR_2$, wobei $R_2$ eine Gruppe ist, die unter den gesättigten oder ungesättigten, geradkettigen, cyclischen oder verzweigten Alkyl- oder Alkoxygruppen mit 1 bis 30 Kohlenstoffatomen, die gegebenenfalls hydroxyliert sind, ausgewählt ist;

- $R_1$ eine Gruppe, die unter den Gruppen der folgenden Formeln (a), (b) und (c) ausgewählt ist:

    a) $-CO-NR_3R_4$
    b) $-CO-O-R_5$
    c) $-SO_2-R_5$

    worin bedeuten:

$R_3$ Wasserstoff oder eine gesättigte oder ungesättigte, geradkettige oder verzweigte $C_{1-6}$-Alkylgruppe, die gegebenenfalls hydroxyliert ist,

$R_4$ Wasserstoff oder eine Gruppe, die unter den gesättigten oder ungesättigten, geradkettigen, cyclischen oder verzweigten Alkylgruppen mit 1 bis 30 Kohlenstoffatomen, die gegebenenfalls hydroxyliert sind, ausgewählt ist,

$R_5$ eine Gruppe, die unter den gesättigten oder ungesättigten, geradkettigen, cyclischen oder verzweigten Alkylgruppen mit 1 bis 30 Kohlenstoffatomen, die gegebenenfalls hydroxyliert sind, ausgewählt ist,

in einer auf die menschliche Haut, die Körperhaare oder die Haare depigmentierend und/oder bleichend wirkenden Zusammensetzung.

2. Verwendung nach Anspruch 1, **dadurch gekennzeichnet, daß** das Aminophenolderivat mindestens eine der folgenden Bedingungen erfüllt:

- R bedeutet Wasserstoff,
- die Gruppe -OR befindet sich an der Phenylgruppe in ortho- oder in para-Stellung,
- $R_1$ ist unter den Gruppen der Formeln (a) oder (b) ausgewählt.

3. Verwendung nach Anspruch 1 oder 2, **dadurch gekennzeichnet, daß** das Aminophenolderivat unter N-Ethyloxycarbonyl-4-aminophenol; N-Ethyloxycarbonyl-O-ethyloxycarbonyl-4-amino-phenol; N-Cholesteryloxycarbonyl-4-amino-phenol und N-Ethylaminocarbonyl-4-amino-phenol ausgewählt ist.

4. Verwendung mindestens eines Aminophenolderivats der Formel (I) nach einem der Ansprüche 1 bis 3 in kosmetischen und/oder dermatologischen Zusammensetzungen oder zur Herstellung von kosmetischen und/oder dermatologischen Zusammensetzungen als Inhibitor der Tyrosinase und/oder Inhibitor der Melaninsynthese.

5. Kosmetische und/oder dermatologische Zusammensetzung, die mindestens ein Aminophenolderivat der Formel (I) nach einem der Ansprüche 1 bis 4 in einem kosmetisch und/oder dermatologisch akzeptablen Medium enthält.

**6.** Zusammensetzung nach dem vorhergehenden Anspruch, **dadurch gekennzeichnet, daß** sie dazu dient, die menschliche Haut zu depigmentieren und/oder zu bleichen und/oder Pigmentflecken der Haut zu entfernen und/ oder das Körperhaar und/oder Haar zu depigmentieren.

**7.** Zusammensetzung nach einem der Ansprüche 5 bis 6, **dadurch gekennzeichnet, daß** das Aminophenolderivat in einer Menge von 0,01 bis 10 % des Gesamtgewichts der Zusammensetzung vorliegt.

**8.** Zusammensetzung nach einem der Ansprüche 5 bis 7, **dadurch gekennzeichnet, daß** sie ferner mindestens einen Wirkstoff enthält, der unter den keratolytischen und/oder abschuppenden Wirkstoffen, entzündungshemmenden Wirkstoffen, reizlindernden Mitteln, weiteren depigmentierenden Wirkstoffen und deren Gemischen ausgewählt ist.

**9.** Aminophenolderivate der folgenden Formel (I):

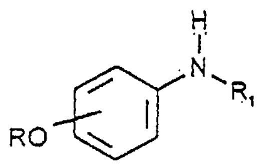

(I)

worin bedeuten:

- R Wasserstoff oder eine Gruppe -$COR_2$, wobei $R_2$ eine Gruppe ist, die unter den gesättigten oder ungesättigten, geradkettigen, cyclischen oder verzweigten Alkyl- oder Alkoxygruppen mit 1 bis 30 Kohlenstoffatomen, die gegebenenfalls hydroxyliert sind, ausgewählt ist;

- $R_1$ eine Gruppe, die unter den folgenden Gruppen ausgewählt ist:

  a) -CO-$NR_3R_4$
  b) -Cholesteryloxycarbonyl
  c) -$SO_2$-$R_5$

  worin bedeuten:

$R_3$ Wasserstoff oder eine gesättigte oder ungesättigte, geradkettige oder verzweigte $C_{1-6}$-Alkylgruppe, die gegebenenfalls hydroxyliert ist,

$R_4$ eine Gruppe, die unter den gesättigten oder ungesättigten, geradkettigen, cyclischen oder verzweigten Alkylgruppen mit 13 bis 30 Kohlenstoffatomen, die gegebenenfalls hydroxyliert sind, ausgewählt ist,

$R_5$ eine Gruppe, die unter den gesättigten oder ungesättigten, geradkettigen, cyclischen oder verzweigten Alkylgruppen mit 1 bis 30 Kohlenstoffatomen, die gegebenenfalls hydroxyliert sind, ausgewählt ist.

**10.** Kosmetisches Verfahren zur Depigmentierung und/oder zum Bleichen der menschlichen Haut, des Körperhaares und/oder des Haares, das **dadurch gekennzeichnet ist, daß** es darin besteht, auf die Haut, das Körperhaar oder das Haar eine Zusammensetzung nach einem der Ansprüche 5 bis 8 aufzutragen.

**11.** Zusammensetzung nach Anspruch 7, **dadurch gekennzeichnet, daß** das Aminophenolderivat in einer Menge von 0,005 bis 5 % des Gesamtgewichts der Zusammensetzung vorliegt.